# EUROPÄISCHE PATENTANMELDUNG

(11) **EP 1 943 948 A1**
(43) Veröffentlichungstag der Anmeldung: **16.07.2008**
(21) Anmeldenummer: 07000658.0
(22) Anmeldetag: 13.01.2007
(51) Int. Cl.: A61B 5/151

(54) **Stechgerät**

(71) Anmelder: Roche Diagnostics GmbH, 68305 Mannheim (DE); F.HOFFMANN-LA ROCHE AG, 4070 Basel (CH)
(72) Erfinder: Konya, Ahmet, 68305 Mannheim (DE)
(74) Vertreter: Mommer, Niels

(57) **Zusammenfassung**

Die Erfindung betrifft ein Stechgerät zum Gewinnen einer Körperflüssigkeitsprobe mit einem Aufnahmefach zum Aufnehmen eines Lanzettenvorratsbands (5) mit mehreren Lanzetten (6), die quer zur Längsrichtung des Lanzettenvorratsbandes (5) angeordnet sind, einer Fördereinrichtung, um durch Transportieren eines in das Aufnahmefach eingesetzten Lanzettenvorratsbandes (5) die Lanzetten (6) des Lanzettenvorratsbands (5) nacheinander in eine Einstichposition zu bringen, und einem Stechantrieb, um eine Lanzette (6), die sich in der Einstichposition befindet, in eine Einstichbewegung zu versetzten. Das Stechgerät zeichnet sich aus durch eine Biegeeinrichtung (10), um einen Abschnitt des Lanzettenvorratsbands (5) in Längsrichtung des Lanzettenvorratsbandes (5) zu biegen und dadurch eine Lanzettenspitze (6a) einer Lanzette (6) für einen Einstich von dem Lanzettenvorratsband (5) zu trennen. Die Erfindung betrifft ferner ein Stechsystem umfassend ein derartiges Stechgerät (1) und ein Lanzettenvorratsband (5) mit mehreren Lanzetten (6) sowie ein Verfahren zum Trennen einer Lanzettenspitze von einem Lanzettenvorratsband (5), wobei das Lanzettenvorratsband (5) in Längsrichtung gebogen wird, so dass sich die Lanzettenspitze (6a) von dem Lanzettenvorratsband (5) abhebt.

## Beschreibung

Die Erfindung betrifft ein Stechgerät zum Gewinnen einer Körperflüssigkeitsprobe mit einem Aufnahmefach zum Aufnehmen eines Lanzettenvorratsbands mit mehreren Lanzetten, die quer zur Längsrichtung des Lanzettenvorratsbandes angeordnet sind und eine Lanzettenspitze zum Erzeugen einer Einstichwunde aufweisen, einer Fördereinrichtung, um durch Transportieren eines in das Aufnahmefach eingesetzten Lanzettenvorratsbandes die Lanzetten des Lanzettenvorratsbands nacheinander in eine Einstichposition zu bringen, und einem Stechantrieb, um eine Lanzette, die sich in der Einstichposition befindet, in eine Einstichbewegung zu versetzten. Die Erfindung betrifft ferner ein Stechsystem umfassend ein derartiges Stechgerät sowie ein Lanzettenvorratsband mit mehreren Lanzetten und ein Verfahren zum Trennen einer Lanzettenspitze von einem Lanzettenvorratsband.

Derartige Stechgeräte und dazugehörende Lanzettenvorratsbänder, die quer zur Längsrichtung des Lanzettenvorratsbandes angeordnete Lanzetten enthalten, sind beispielsweise aus der US 2003/0199906 A1 und der DE 2803345 B1 bekannt. Die in diesen Veröffentlichungen beschriebenen Lanzettenvorratsbänder umfassen einen flexiblen Träger, auf dem die Lanzetten angeordnet sind und einen die Lanzetten bedeckenden Sterilschutz. Der Sterilschutz der bekannten Lanzettenvorratsbänder wird von einer Folie gebildet, die abgezogen werden muss, bevor die Lanzetten für einen Einstich verwendet werden können. Zum Entfernen des Sterilschutzes ist bei bekannten Stechsystemen ein relativ hoher Geräteaufwand erforderlich.

Aufgabe der Erfindung ist es deshalb, einen Weg aufzuzeigen, wie bei einem Stechgerät der Eingangs genannten Art mit geringerem Aufwand Lanzetten eines Lanzettenvorratsbandes für einen Einstich bereit gestellt werden können.

Die Aufgabe wird erfindungsgemäß gelöst durch eine Biegeeinrichtung, um einen Abschnitt des Lanzettenvorratsbands in Längsrichtung des Lanzettenvorratsbandes zu biegen und dadurch eine Lanzettenspitze einer Lanzette für einen Einstich von dem Lanzettenvorratsband zu trennen.

Im Rahmen der Anmeldung wird unter einer Lanzettenspitze das vordere, zum Einstich bestimmte Ende einer Lanzette verstanden.

Wird ein Lanzettenvorratsband, das mehrere quer zur Längsrichtung des Lanzettenvorratsbandes angeordnete Lanzetten enthält, in Längsrichtung gebogen, so behält eine Lanzette in dem gebogenen Abschnitt des Lanzettenvorratsbands ihre Orientierung weitgehend bei. Dies führt dazu, dass sich die Spitze der Lanzette von dem Vorratsband abhebt und durch eine Sterilschutz- oder Trägerschicht hindurch bricht. Auf diese Weise kann eine Lanzettenspitze einer Lanzette für einen Einstich von dem Lanzettenvorratsband getrennt werden, so dass die Lanzette für einen Einstich bereit steht. Es ist nicht erforderlich, dass eine Lanzette durch das Biegen des Lanzettenvorratsbandes vollständig von dem Lanzettenvorratsband getrennt wird. Es genügt, wenn durch das Biegen die Lanzettenspitze, die bei einem Einstich in die Haut eines an das Stechgerät angelegten Körperteils eindringt, von dem Lanzettenvorratsband getrennt wird. Durch das Biegen wird ein vorderer Lanzettenbereich, zu dem die Lanzettenspitze gehört, von dem Lanzettenvorratsband getrennt.

Ein erfindungsgemäßes Stechgerät und ein Lanzettenvorratsband mit mehreren Lanzetten, die quer zur Längsrichtung des Lanzettenvorratsbandes angeordnet sind, bilden ein Stechsystem. Ein weiterer Aspekt der Erfindung betrifft ein Verfahren zum Trennen einer Lanzettenspitze von einem Lanzettenvorratsband, wobei das Lanzettenvorratsband in Längsrichtung gebogen wird, so dass sich die Lanzettenspitze von dem Lanzettenvorratsband abhebt.

Das Lanzettenvorratsband eines erfindungsgemäßen Stechsystems kann beispielsweise ähnlich wie aus der US 2003/0199906 A1 oder der DE 2803345 B1 bekannte Lanzettenvorratsbänder ausgebildet sein und eine flexible Trägerschicht, auf der die Lanzetten angeordnet sind und einen die Lanzetten bedeckenden Sterilschutz umfassen. Der Sterilschutz kann beispielsweise als Folie, insbesondere aus Kunststoff oder Papier, oder als Silikonschicht ausgebildet sein. Eine weitere Möglichkeit für das Lanzettenvorratsband besteht beispielsweise darin, Lanzetten auf einem Trägerband, beispielsweise aus Papier, Kunststoff- oder/und Metallfolie, anzuordnen und das Trägerband zu falten, so dass es die Lanzettenspitzen bedeckt und auf diese Weise vor schädlichen Umwelteinflüssen schützt. Prinzipiell ist es auch möglich, die Lanzetten auf einem Trägerband anzuordnen, das aufgewickelt wird, so dass die Lanzetten in einer so gebildeten Lanzettenvorratsbandrolle zwischen der Oberseite und der Unterseite des Trägerbands vor schädlichen Umwelteinflüssen geschützt sind.

Weitere Einzelheiten und Vorteile der Erfindung werden anhand eines Ausführungsbeispiels unter Bezugnahme auf die beigefügten Zeichnungen erläutert. Die dabei beschriebenen Merkmale können einzeln oder in Kombination zum Gegenstand von Ansprüchen gemacht werden. Es zeigen:
- Fig. 1: ein Ausführungsbeispiel eines erfindungsgemäßen Stechgeräts;
- Fig. 2: eine schematische Innenansicht des in Figur 1 dargestellten Ausführungsbeispiels mit einem eingelegten Lanzettenvorratsband vor einem Biegevorgang;
- Fig. 3: eine um 90° gedrehte Ansicht zur Figur 2;
- Fig. 4: eine Darstellung gemäß Figur 2 nach dem Biegen des Lanzettenvorratsbandes;
- Fig. 5: eine um 90° gedrehte Ansicht zu Figur 4;
- Fig. 6: die Position des Lanzettenvorratsbandes hinsichtlich der Biegeeinrichtung nach einem Einstich;
- Fig. 7: eine um 90° gedrehte Ansicht zu Figur 6;
- Fig. 8: das Lanzettenvorratsband in einer Probenaufnahmeposition; und
- Fig. 9: eine um 90° gedrehte Ansicht zu Figur 8.

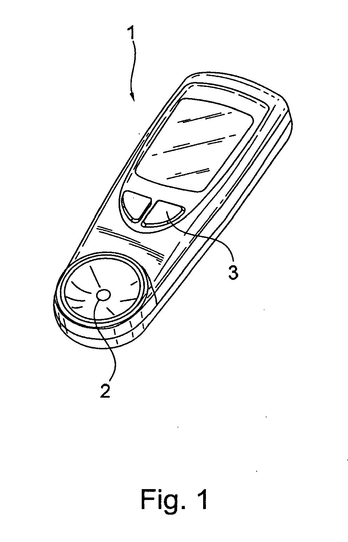

Figur 1 zeigt in einer schematischen Darstellung ein Ausführungsbeispiel eines erfindungsgemäßen Stechgeräts 1 zum Gewinnen einer Körperflüssigkeitsprobe. Das Stechgerät 1 weist eine Geräteöffnung 2 auf, gegen die ein Körperteil zum Erzeugen einer Einstichwunde gepresst wird. Das Stechgerät 1 umfasst ferner Bedienungselemente 3 in Form von Tasten und eine Anzeigeeinrichtung 4 in Form einer Flüssigkristallanzeige zum Anzeigen von Untersuchungsergebnissen. Das Stechgerät 1 hat ein Aufnahmefach (nicht dargestellt) für ein Lanzettenvorratsband. Das Aufnahmefach hat eine verschließbare Öffnung, die sich auf der Rückseite des in Figur 1 dargestellten Ausführungsbeispiels befindet.

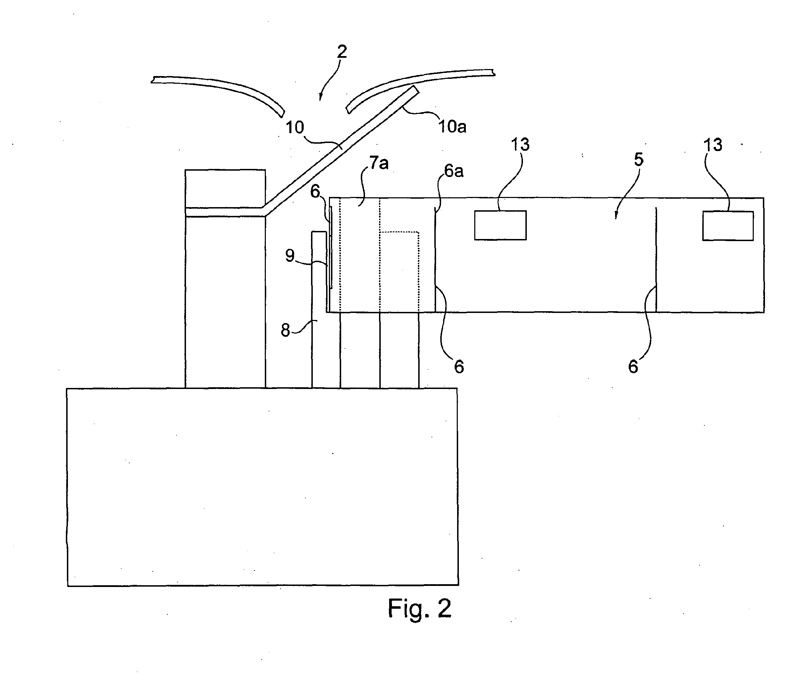

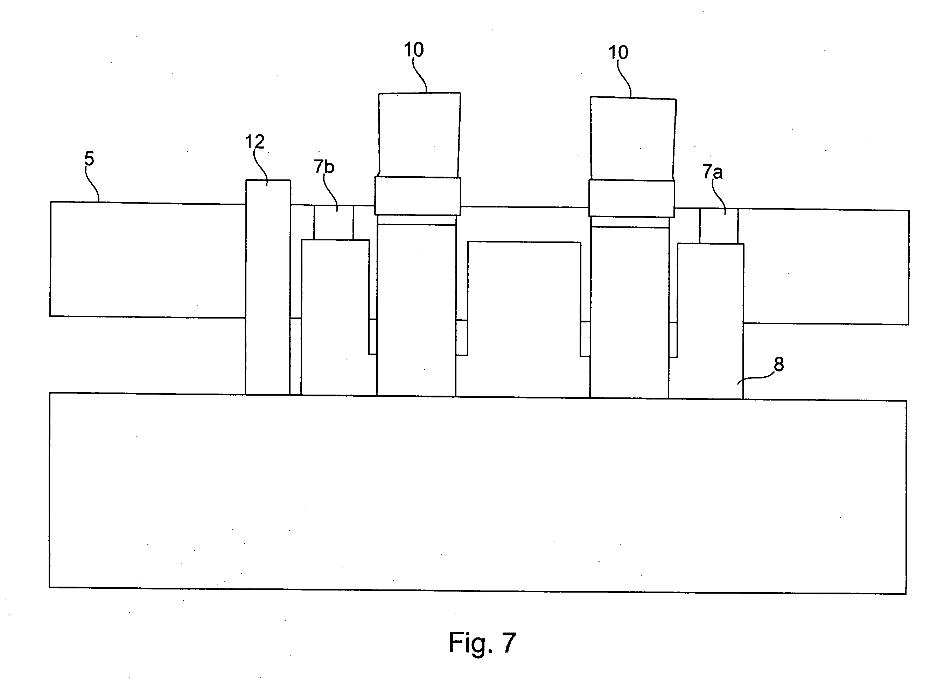

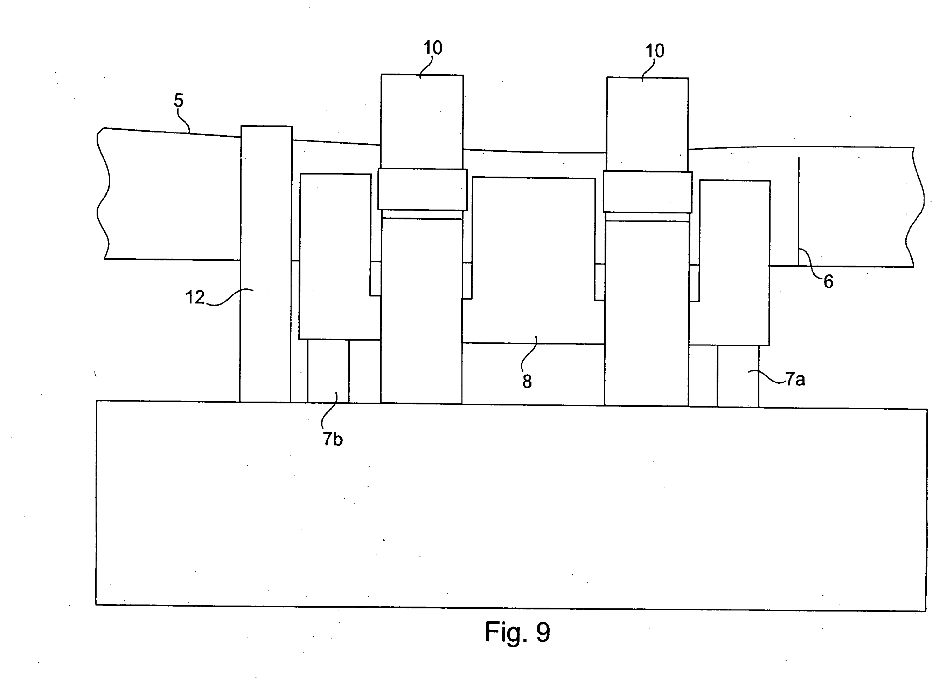

Das in Figur 1 dargestellte Stechgerät 1 bildet zusammen mit dem in den Figuren 2 bis 9 gezeigten Lanzettenvorratsband 5 ein Stechsystem. Das Lanzettenvorratsband 5 trägt mehrere Lanzetten 6, die quer zur Längsrichtung des Lanzettenvorratsbandes 5 angeordnet sind und jeweils eine Lanzettenspitze 6a zum Erzeugen einer Einstichwunde aufweisen. Die Figuren 2 bis 9 zeigen in einer schematischen Darstellung einen Ausschnitt des Geräteinneren mit einem in das Stechgerät 1 eingelegten Lanzettenvorratsband 5 in verschiedenen Betriebspositionen, anhand von denen im Folgenden die Arbeitsweise des Stechgeräts 1 erläutert wird.

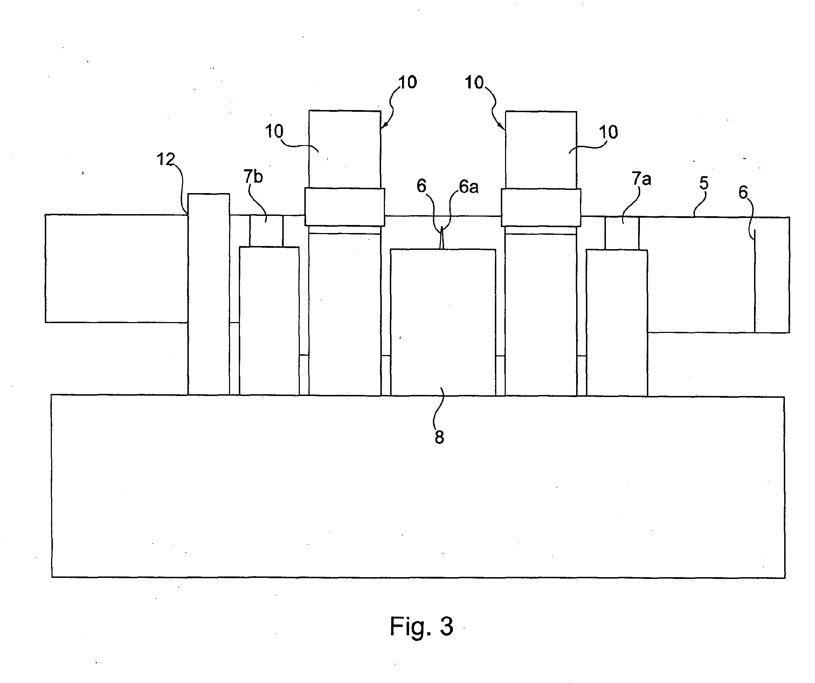

Die Lanzetten 6 eines in das Stechgerät 1 eingelegten Lanzettenvorratsbandes 5 können durch eine Fördereinrichtung nacheinander in eine Einstichposition gebracht werden. Eine Lanzette 6, die sich in der Einstichposition befindet, zeigt mit ihrer Lanzettenspitze 6a vom Inneren des Stechgeräts 1 hin zu der Geräteöffnung 2. In den Figuren 2 und 3 ist das Lanzettenvorratsband 5 so positioniert, dass sich eine Lanzette 6 in der Einstichposition befindet.

Die Fördereinrichtung kann beispielsweise eine Wickeleinrichtung sein, mit der ein Lanzettenvorratsband 5, das beispielsweise aufgewickelt oder zickzackförmig zu einem Stapel gefaltet in das Aufnahmefach des Stechgeräts 1 eingesetzt sein kann, nach Gebrauch aufgewickelt und auf diese Weise in Längsrichtung bewegt werden kann.

Eine Lanzette 6, die sich in Einstichposition befindet, also mit ihrer Lanzettenspitze 6a zu der Geräteöffnung 2 zeigt, wie es in Figur 2 angedeutet ist, kann mit dem Stechantrieb 8 in eine Einstichbewegung versetzt werden. Der Stechantrieb 8 umfasst bei dem dargestellten Ausführungsbeispiel eine Halterung mit einem Schlitz 9, durch den das Lanzettenvorratsband 5 hindurch geführt ist. Bei einem Einstich wird der Stechantrieb 8 mit der Halterung in Einstichrichtung bewegt und dabei der Abschnitt des Lanzettenvorratsbandes 5, der sich in dem Schlitz 9 befindet, zusammen mit einer sich in Einstichposition befindenden Lanzette 6 beschleunigt, so dass die Lanzette 6 mit ihrer Spitze 6a in ein gegen die Geräteöffnung 2 gepresstes Körperteil einsticht. Bei einem Einstich bleibt die Lanzette 6 mit dem Lanzettenvorratsband 5 verbunden, da sich bei dem Biegevorgang nur die Lanzettenspitze 6a von dem Lanzettenvorratsband 5 abhebt.

Eine Einstichbewegung der Halterung des Stechantriebs 8 kann sich im einfachsten Fall auf das Lanzettenvorratsband 5 übertragen, indem das Lanzettenvorratsband 5 mit seiner Längskante, die von den Lanzettenspitze 6a des Lanzettenvorratsbandes 5 abgewandt ist, auf der Halterung an einem Boden des Schlitzes 9 aufliegt. Eine verbesserte Übertragung einer Einstich- und Rückführbewegung des Stechantriebs 8 auf das Lanzettenvorratsband 5 kann man dadurch erreichen, dass der Schlitz 9 durch eine geeignete Mechanik verengt wird, sobald sich eine Lanzette 6 in Einstichposition befindet, und das Lanzettenvorratsband 5 auf diese Weise in dem Schlitz 9 eingeklemmt wird. Nach abgeschlossener Rückführbewegung wird der Schlitz 9 wieder verbreitert, so dass das Lanzettenvorratsband 5 von der Fördereinrichtung weiterbewegt werden kann.

In Förderrichtung vor dem Schlitz 9 ist bei dem dargestellten Ausführungsbeispiel eine Umlenkrolle 7a angeordnet, die das Lanzettenvorratsband 5 umlenkt. Auf diese Weise kann der Transport des Lanzettenvorratsbandes 5 durch den Schlitz 9 hindurch erleichtert und ein Blockieren des Lanzettenvorratsbandes 5 in dem Schlitz 9 verhindert werden. In Förderrichtung hinter dem Schlitz 9 ist eine Umlenkrolle 7b angeordnet, die ebenso wie die Umlenkrolle 7a den Transport des Lanzettenvorratsbandes 5 durch den Schlitz 9 hindurch unterstützt.

Die Spitzen 6a der Lanzetten 6 des eingesetzten Lanzettenvorratsbandes 5 sind von einem Sterilschutz umgeben, der bei dem dargestellten Ausführungsbeispiel als eine Kunststofffolie ausgebildet ist, welche die auf einem flexiblen Trägerband angeordneten Lanzetten 6 bedeckt. Das Trägerband kann ebenso wie der Sterilschutz beispielsweise aus Kunststoff, Papier oder Metall gefertigt werden.

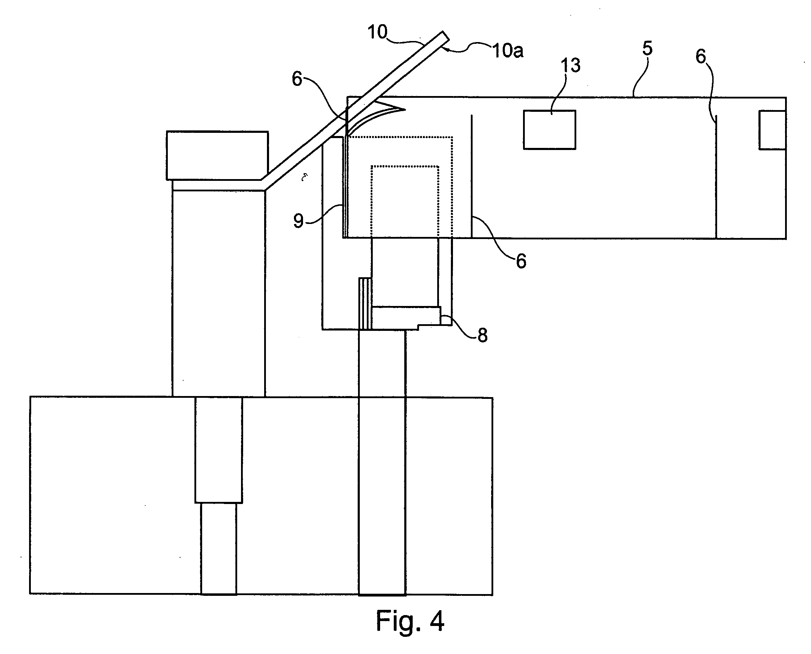

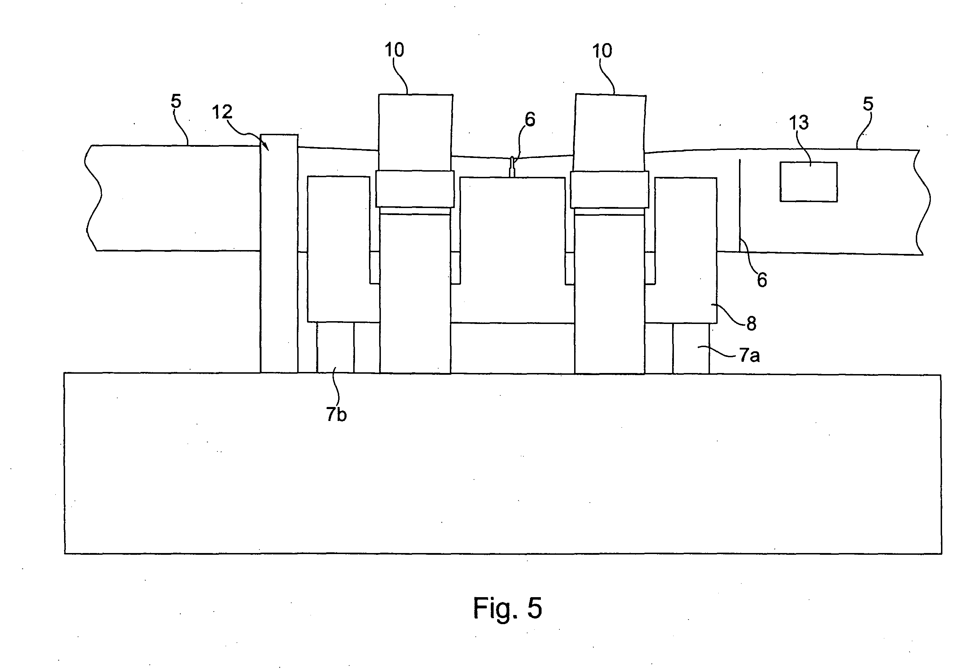

Die Lanzettenspitze 6a einer Lanzette 6 wird von dem Lanzettenvorratsband 5 getrennt, indem eine Biegeeinrichtung 10 das Lanzettenvorratsband 5 in Längsrichtung des Lanzettenvorratsbandes 5 biegt. Hiefür wird das Lanzettenvorratsband 5 von dem Stechantrieb 8 in Einstichrichtung bewegt, so dass das Lanzettenvorratsband 5 mit seiner in Einstichrichtung vorderen Längskante an die Biegeeinrichtung 10 stößt. In den Figuren 4 und 5 ist ein in Längsrichtung gebogener Abschnitt des Lanzettevorratsbandes 5 mit dem in Einstichrichtung verfahrenen Stechantrieb 8 dargestellt.

Bei einer derartigen Biegung des Lanzettenvorratsbandes 5 behält die Lanzette 6 ihre Orientierung bei und reißt deshalb den sie bedeckenden Sterilschutz, bei dem dargestellten Ausführungsbeispiel eine Deckfolie, oder das flexible Trägerband auf. Die Lanzettenspitze 6a hebt sich bei dem Biegevorgang von dem Lanzettenvorratsband 5 ab und wird auf diese Weise von ihm getrennt, so dass mit der Lanzettenspitze 6a zum Gewinnen einer Körperflüssigkeitsprobe in ein gegen die Geräteöffnung 2 gepresstes Körperteil eingestochen werden kann.

Die Biegeeinrichtung 10 wirkt zum Biegen des Lanzettenvorratsbandes 5 auf eine Längskante des Lanzettenvorratsbandes 5 ein. Bei dem dargestellten Ausführungsbeispiel wird das Lanzettenvorratsband 5 aus der in den Figuren 2 und 3 dargestellten Position in Einstichrichtung bewegt, bis es in die in den Figuren 4 und 5 dargestellte Position gelangt, in der ein Abschnitt des Lanzettenvorratsbands 5 in Längsrichtung gebogen wird, indem eine Längskante des Lanzettenvorratsbandes 5 von dem Stechantrieb 8 gegen die Biegeeinrichtung 10 gedrückt wird. Das Biegen des Lanzettenvorratsbandes 5 in Längsrichtung und damit das Bereitmachen einer Lanzette 6 für einen Einstich erfolgt bei dem dargestellten Ausführungsbeispiel in einer frühen Phase einer Einstichbewegung. Auf diese Weise kann die Gefahr einer Verschmutzung der Lanzettenspitze 6a, die nach dem Trennen der Lanzettenspitze 6a von dem Lanzettenvorratsband 5 prinzipiell wegen des Durchbrochenen Sterilschutzes besteht, minimiert.

Prinzipiell ist es ausreichend, mit einer Biegeeinrichtung 10 an einer Stelle, beispielsweise neben einer Lanzette 6 auf eine Längskante des Lanzettenvorratsbandes 5 einzuwirken. Eine bessere Biegung lässt sich jedoch erreichen, wenn die Biegeeinrichtung 10 an zwei voneinander beabstandeten Stellen auf die den Lanzettenspitzen 6a zugewandte Längskante des Lanzettenvorratsbandes 5 einwirkt, so dass zwischen diesen beiden Stellen ein Bandschnitt gebogen, der keinen Kontakt zu der Biegeeinrichtung 10 hat und eine in Lanzettenspitze 6a, die sich diesem Abschnitt des Lanzettenvorratsbandes 5 befindet, die Biegeeinrichtung 10 nicht berührt. Dies lässt sich beispielsweise dadurch erreichen, dass die Biegeeinrichtung 10 einen Schlitz aufweist. Auf diese Weise kann eine Lanzettenspitze 6a beim Biegen des Lanzettenvorratsbandes 5 ihre Orientierung beibehalten und in den Schlitz hineinragen. In Figur 5 ist dargestellt, wie die Biegeeinrichtung 10 links und rechts neben der Lanzette 6 auf eine Längskante des Lanzettenvorratsbandes 5 drückt.

Bei dem dargestellten Ausführungsbeispiel hat die Biegeeinrichtung 10 zwei Biegeflächen 10a, die sich in einem Abstand voneinander schräg zur Einstichrichtung erstrecken. Wie insbesondere die Figuren 2 und 4 zeigen, können derartige Biegeflächen 10a gabelartig, beispielsweise als Stege, ausgebildet sein, die bei dem dargestellten Ausführungsbeispiel geradlinig verlaufen. Prinzipiell können die Biegeflächen 10a jedoch auch gekrümmt sein. Es genügt, wenn eine Längskante des Lanzettenvorratsbandes 5, die gegen eine Biegefläche 10a gedrückt wird, abgleitet, so dass der entsprechende Abschnitt des Lanzettenvorratsbandes 5 in Längsrichtung abgebogen wird.

Das in Figur 1 gezeigte Ausführungsbeispiel weist eine Testeinrichtung 12 zum Untersuchen einer Körperflüssigkeitsprobe auf. Eine derartige Testeinrichtung 12 kann eine photometrische Testeinrichtung sein, mit der eine Verfärbung eines Testfeldes untersucht wird, das Nachweisreagenzien enthält, die eine von der Analytkonzentration abhängende Verfärbung des Testfeldes bewirken. Beispielsweise kann die Testeinrichtung 12 auch eine elektrochemische Testeinrichtung sein, mit der ein von der Analytkonzentration abhängender Messstrom erfasst wird. Geeignete Testeinrichtungen sind beispielsweise in handelsübliche Analysehandgeräte integriert, mit denen beispielsweise Diabetiker ihren Blutzuckergehalt überwachen. Das Lanzettenvorratsband 5 trägt neben Lanzetten 6 auch Testfelder 13, so dass mit dem beschriebenen Stechsystem eine Körperflüssigkeitsprobe gewonnen und anschließend, beispielsweise zur Messung des Glukosegehalts, untersucht werden kann.

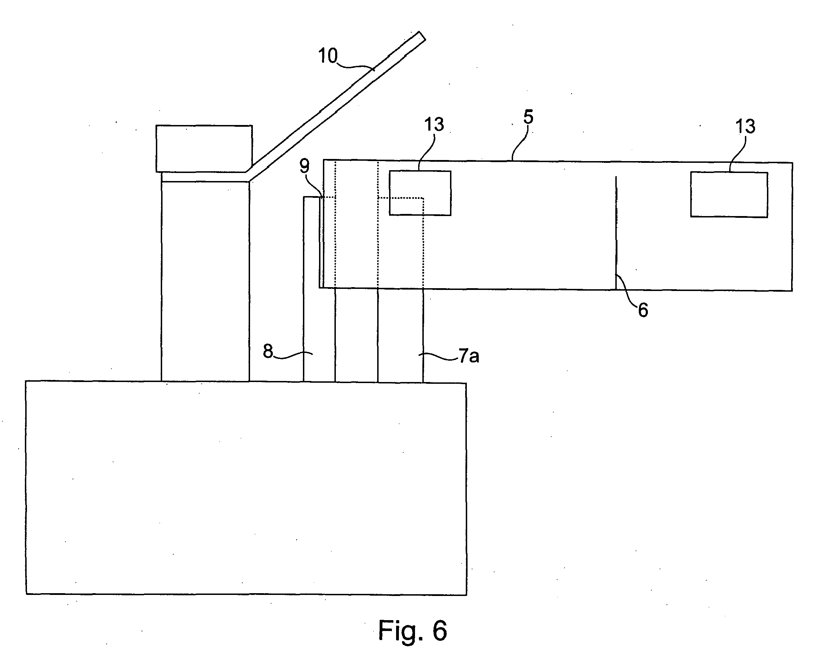

Die Testeinrichtung 12 des Stechgeräts 1 wirkt mit einem Testfeld 13 des Lanzettenvorratsbandes 5 zusammen, das eine aus einer erzeugten Einstichwunde austretende Körperflüssigkeitsprobe aufnimmt. Dies wird im Folgenden anhand der Figuren 6 bis 9 erläutert.

Die mit einer Einstichbewegung begonnene Einwirkung des Biegemechanismus 10 auf das Lanzettenvorratsband 5 wird durch eine anschließende Rückführbewegung beendet, bei welcher der Lanzettenantrieb 8 und der in dem Schlitz 9 angeordnete Abschnitt des Lanzettenvorratsbandes 5 wieder in ihre in den Figuren 2 und 6 dargestellte Ausgangsposition zurückkehren, so dass die Biegeeinrichtung 10 nicht mehr gegen das Lanzettenvorratsband 5 drückt. Zum Aufnehmen einer Körperflüssigkeitsprobe wird so die Einwirkung des Biegemechanismus 10 auf das Lanzettenvorratsband 5 nach einem Einstich unterbrochen, so dass der gebogene Lanzettenvorratsbandabschnitt wieder in seine flache Lage zurückkehrt, in der er beispielsweise in Figur 6 dargestellt ist.

Anschließend wird das Lanzettenvorratsband 5 von der Fördereinrichtung weiterbewegt, so dass ein Testfeld 13 des Lanzettenvorratsbandes 5 mit der Geräteöffnung 2 und einer zuvor erzeugten Einstichwunde eines gegen die Geräteöffnung 2 gepressten Körperteils fluchtet. Danach wird das Lanzettenvorratsband 5 von dem Stechantrieb 8 erneut in Einstichrichtung bewegt und so von dem Biegemechanismus 10 erneut in Längsrichtung gebogen. Dabei wird das Lanzettenvorratsband 5 mit einer Teilfläche an das Körperteil angelegt, in dem die Einstichwunde erzeugt wurde. Bei dem dargestellten Ausführungsbeispiel enthält die Teilfläche, mit der das Lanzettenvorratsband 5 an das Körperteil angelegt wird, ein Testfeld 13. Das Lanzettenvorratsband 5 wird in diesem Arbeitsschritt also erneut mit seiner Längskante gegen die Biegeeinrichtung 10 gedrückt.

Indem eine Körperflüssigkeitsprobe durch Anlegen einer Teilfläche des in Längsrichtung gebogenen Lanzettenvorratsbandes an ein Körperteil, in dem eine Einstichwunde erzeugt wurde, aufgenommen wird, kann eine Verschmutzung des Stechgeräts vorteilhaft vermieden werden. Verwendete Lanzetten und untersuchte Körperflüssigkeitsproben können zusammen mit einem aufgebrauchten Lanzettenvorratsband hygienisch und benutzerfreundliche entsorgt werden.

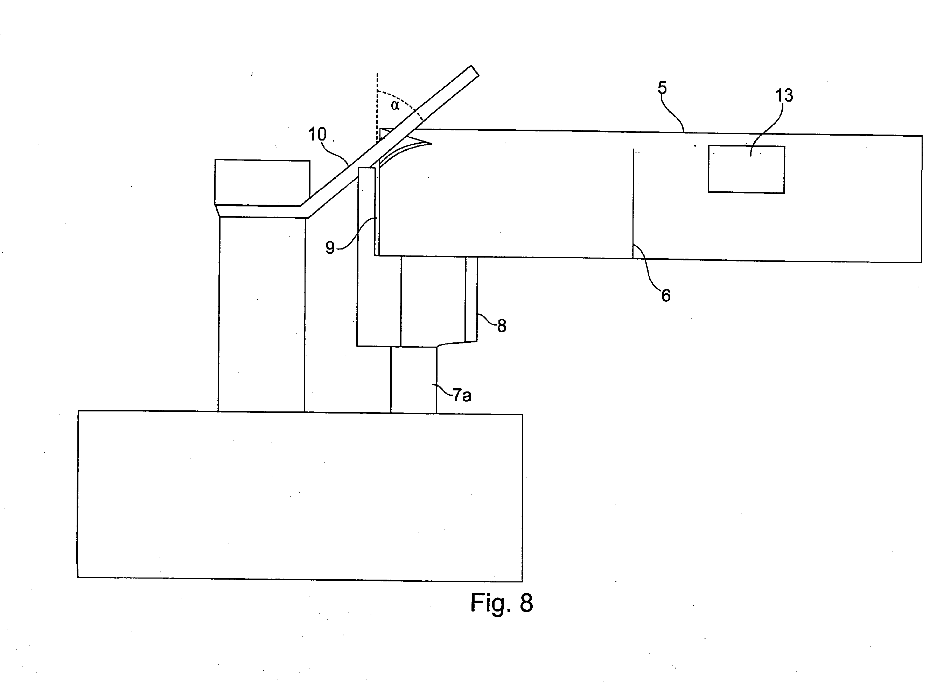

Von der Biegeeinrichtung 10 wird das Lanzettenvorratsband 5 bei dem dargestellten Ausführungsbeispiel um einen in Figur 8 dargestellten Winkel α von etwa 45° in Längsrichtung gebogen. Das Lanzettenvorratsband wird bei dem dargestellten Ausführungsbeispiel um mindestens 25°, insbesondere mindestens 30° gebogen. Besonders günstig zur Aufnahme einer Körperflüssigkeitsprobe sind Biegewinkel von 40° bis 90°, so dass eine vorteilhaft große Teilfläche des Lanzettenvorratsbandes 5 zum Aufnehmen einer Körperflüssigkeitsprobe an das betreffende Körperteil angelegt werden kann.

Nach dem Aufnehmen einer Körperflüssigkeitsprobe wird das Lanzettenvorratsband 5 von dem Stechantrieb 8 wieder zurückbewegt, also erneut von der Biegeeinrichtung 10 getrennt und somit die Einwirkung der Biegeeinrichtung 10 erneut unterbrochen, so dass der gebogene Lanzettenvorratsbandabschnitt wieder in seine flache Lage zurückkehrt. Anschließend wird das Lanzettenvorratsband 5 von der Fördereinrichtung einen Schritt weiter transportiert, um das mit einer Körperflüssigkeitsprobe benetzte Testfeld 13 zu der Testeinrichtung 12 zu befördern. Die Testeinrichtung 12 ermittelt dann in Zusammenwirkung mit dem Testfeld 13 eine Analytkonzentration, beispielsweise die Glukosekonzentration, der Körperflüssigkeit.

### Bezugszeichenliste

- 1: Stechgerät
- 2: Geräteöffnung
- 3: Bedienungselemente
- 4: Zeigeeinrichtung
- 5: Lanzettenvorratsband
- 6: Lanzetten
- 6a: Lanzettenspitze
- 7a: Umlenkrolle
- 7b: Umlenkrolle
- 8: Stechantrieb

- 10: Biegeeinrichtung
- 10a: Biegefläche

- 12: Testeinrichtung
- 13: Testfeld

## Patentansprüche

1. Stechgerät zum Gewinnen einer Körperflüssigkeitsprobe mit
einem Aufnahmefach zum Aufnehmen eines Lanzettenvorratsbands (5) mit mehreren Lanzetten (6), die quer zur Längsrichtung des Lanzettenvorratsbandes (5) angeordnet sind und eine Lanzettenspitze (6a) zum Erzeugen einer Einstichwunde aufweisen,
einer Fördereinrichtung, um durch Transportieren eines in das Aufnahmefach eingesetzten Lanzettenvorratsbandes (5) die Lanzetten (6) des Lanzettenvorratsbands (5) nacheinander in eine Einstichposition zu bringen, und
einem Stechantrieb (8), um eine Lanzette (6), die sich in der Einstichposition befindet, in eine Einstichbewegung zu versetzten,
**gekennzeichnet durch**
eine Biegeeinrichtung (10), um einen Abschnitt des Lanzettenvorratsbands (5) in Längsrichtung des Lanzettenvorratsbandes (5) zu biegen und **dadurch** eine Lanzettenspitze (6a) einer Lanzette (6) für einen Einstich von dem Lanzettenvorratsband (5) zu trennen.

2. Stechgerät nach Anspruch 1, **dadurch gekennzeichnet, dass** die Biegeeinrichtung (10) zum Biegen des Lanzettenvorratsbandes (5) auf eine Längskante des Lanzettenvorratsbandes (5) einwirkt.

3. Stechgerät nach Anspruch 2, **dadurch gekennzeichnet, dass** die Biegeeinrichtung (10) zum Biegen des Lanzettenvorratsbandes (5) mit mindestens einer sich schräg zur Einstichrichtung erstreckenden Biegefläche (10a) auf eine Längskante des Lanzettenvorratsbandes (5) einwirkt.

4. Stechgerät nach Anspruch 2 oder 3, **dadurch gekennzeichnet, dass** die Biegeeinrichtung (10) mit zwei Biegeflächen (10a) auf eine Längskante des Lanzettenvorratsbands (5) einwirkt, wobei die Biegeflächen (10a) in einem Abstand von einander angeordnet sind.

5. Stechgerät nach einem der vorhergehenden Ansprüche, **dadurch gekennzeichnet, dass** der zu biegende Abschnitt des Lanzettenvorratsbands (5) zum Biegen in Einstichrichtung bewegt wird.

6. Stechgerät nach einem der vorhergehenden Ansprüche, **dadurch gekennzeichnet, dass** der Stechantrieb (8) für einen Einstich einen Abschnitt des Lanzettenvorratsbandes (5) zusammen mit einer Lanzette (6) in Einstichrichtung bewegt.

7. Stechgerät nach einem der vorhergehenden Ansprüche, **gekennzeichnet durch** eine Testeinrichtung (12) zum Untersuchen einer Körperflüssigkeitsprobe, die **durch** Anlegen einer Teilfläche des in Längsrichtung gebogenen Lanzettenvorratsbandes (5) an ein Körperteil, in dem eine Einstichwunde erzeugt wurde, aufgenommen wird.

8. Stechgerät nach Anspruch 7, **dadurch gekennzeichnet, dass** die Einwirkung der Biegeeinrichtung (10) auf das Lanzettenvorratsband (5) nach einem Einstich unterbrochen, das Lanzettenvorratsband (5) von der Fördereinrichtung weiterbewegt, anschließend von der Biegeeinrichtung (10) erneut in Längsrichtung gebogen und das Lanzettenvorratsband (5) mit einer Teilfläche an das Körperteil angelegt wird, in dem die Einstichwunde erzeugt wurde.

9. Stechsystem zum Gewinnen einer Körperflüssigkeitsprobe umfassend
ein Stechgerät (1) nach einem der vorhergehenden Ansprüche und
ein Lanzettenvorratsband (5) mit mehreren Lanzetten (6), die quer zur Längsrichtung des Lanzettenvorratsbandes (5) angeordnet sind.

10. Stechsystem zum Gewinnen einer Körperflüssigkeitsprobe nach Anspruch 9, **dadurch gekennzeichnet, dass** das Lanzettenvorratsband (5) Testfelder (13) zur Untersuchung von Körperflüssigkeitsproben umfasst.

11. Stechsystem zum Gewinnen einer Körperflüssigkeitsprobe nach Anspruch 9 oder 10, **dadurch gekennzeichnet, dass** die Lanzettenspitzen (6a) der Lanzetten (6) des Lanzettenvorratsbandes (5) von einem Sterilschutz umgeben sind, der bei dem Biegen eines eine Lanzette (6) enthaltenden Abschnitts des Lanzettenvorratsbandes (5) von einer Lanzettenspitze (6a) durchbrochen wird.

12. Verfahren zum Trennen einer Lanzettenspitze (6a) von einem Lanzettenvorratsband (5), wobei das Lanzettenvorratsband (5) in Längsrichtung gebogen wird, so dass sich die Lanzettenspitze (6a) von dem Lanzettenvorratsband (5) abhebt.
